# EUROPÄISCHE PATENTSCHRIFT

(11) **EP 0 478 994 B1**
(45) Veröffentlichungstag und Bekanntmachung des Hinweises auf die Patenterteilung: **05.07.1995**
(21) Anmeldenummer: 91115238.7
(22) Anmeldetag: 10.09.1991
(51) Int. Cl.: C07D 239/06

(54) **Verfahren zur Herstellung von 5-Hydroxy-3,4,5,6-tetrahydro-pyrimidin-Derivaten**
Process for the preparation of 5-hydroxy-3,4,5,6,-tetra hydro-pyrimidine derivatives
Procédé pour la préparation de dérivés de l'hydroxy-5 tétrahydro-3,4,5,6,pyrimidine

(30) Priorität: 22.09.1990 DE 4030059
(43) Veröffentlichungstag der Anmeldung: 08.04.1992
(73) Patentinhaber: BAYER AG, 51368 Leverkusen (DE)
(72) Erfinder: Lantzsch, Reinhard, Dr., W-5600 Wuppertal (DE); Seifert, Hermann, Dr., W-5060 Bergisch Gladbach 2 (DE)

(56) Entgegenhaltungen:
- EP-A- 0 320 796
- GB-A- 1 403 732

## Beschreibung

Die Erfindung betrifft ein neues Verfahren zur Herstellung von bekannten 5-Hydroxy-3,4,5,6-tetrahydro-pyrimi-din-Derivaten, welche als Zwischenprodukte für die Synthese von Insektiziden verwendet werden können.

Es ist bekannt, daß man bestimmte 5-Hydroxy-3,4,5,6-tetrahydro-pyrimidin-Derivate, wie z. B. 2-tert-Butyl-5-hydroxy-3,4,5,6-tetrahydro-pyrimidin-Hydrochlorid, erhält, wenn man entsprechende Amidine (oder deren Säureaddukte), wie z. B. Pivalamidin-Hydrochlorid, mit 1,3-Diamino-2-propanol in Gegenwart von Verdünnungsmitteln, wie z. B. Ethanol, umsetzt (vergleiche EP-A 320 796). In der GB-A 1 403 732 werden bestimmte Tetrahydropyrimidin-Derivate und ihre Herstellung aus Carbonsäurederivaten und Diaminen beschrieben.

Ein schwerwiegender Nachteil der bekannten Synthesewege ist, daß hierbei 1,3-Diamino-2-propanol als Ausgangsstoff benötigt wird, welcher aus Epichlorhydrin und Ammoniak bisher nur in unbefriedigender Ausbeute (maximal ca. 55 % der Theorie) hergestellt werden kann (vergleiche EP-A 359 956). Die Herstellung von 1,3-Diamino-2-propanol wird zudem in einer für technische Prozesse relativ hohen Verdünnung durchgeführt. Aufarbeitung, Isolierung und Reinigung des Produktes führen so zu sehr hohen Herstellungskosten, die ein solches Verfahren unwirtschaftlich machen, da große Abwassermengen erzeugt werden und große Mengen Ammoniak und Wasser destillativ entfernt werden müssen.

Es wurde nun gefunden, daß man 5-Hydroxy-3,4,5,6-tetra-hydro-pyrimidin-Derivate der allgemeinen Formel (I) 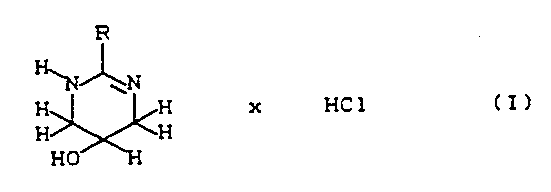
in welcher
- R: für Alkyl mit 1 bis 20 Kohlenstoffatomen steht, das einen oder mehrere, gleiche oder verschiedene Substituenten aus der Reihe der Halogene enthalten kann,
erhält, wenn man Amidine der allgemeinen Formel (II) 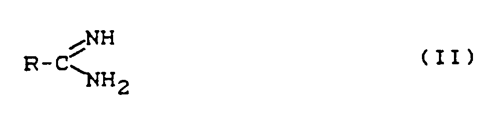
in welcher
- R: die oben angegebene Bedeutung hat,
mit Epichlorhydrin der Formel (III) 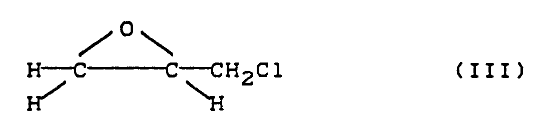
gegebenenfalls in Gegenwart eines Verdünnungsmittels bei Temperaturen zwischen 0°C und 150°C umsetzt.

Der selektive Ablauf des erfindungsgemäßen Verfahrens ist als überraschend anzusehen, da zu erwarten war, daß Amidine mit Epichlorhydrin an beiden Stickstoffatomen reagieren würden. Auch die Bildung von Polymeren, wie aus Umsetzungen von Epichlorhydrin mit Diaminen bekannt, war zu erwarten, Schließlich müßte auch mit einem Ringschluß zu fünfgliedrigen Cyclen (Hydroxymethylimidazolinen) gerechnet werden.

Nach dem erfindungsgemäßen Verfahren können die Verbindungen der Formel (I) im Vergleich mit der bekannten Methode wesentlich einfacher und auf Basis gut zugänglicher Ausgangsstoffe in sehr guten Ausbeuten und in hoher Reinheit hergestellt werden. Das erfindungsgemäße Verfahren stellt somit eine wertvolle Bereicherung des Standes der Technik dar.

In den Formeln (I) und (II) steht Alkyl (R) für geradkettiges oder verzweigtes Alkyl mit 1 bis 20, vorzugsweise 1 bis 10, insbesondere 1 bis 5 Kohlenstoffatomen. Beispielhaft seien Methyl, Ethyl, n-Propyl, i-Propyl, n-Butyl, i-Butyl, s-Butyl, t-Butyl, n-Pentyl, i-Pentyl, s-Pentyl und t-Pentyl genannt.

Alkyl (R) kann einen oder mehrere, vorzugsweise 1 bis 3, gleiche oder verschiedene Substituenten enthalten. Substituenten aus der Reihe der Halogene, wie Fluor, Chlor oder Brom, insbesondere Fluor und Chlor, werden bevorzugt. Vorzugsweise ist Alkyl (R) unsubstituiert.

Vorzugsweise bedeutet R in den allgemeinen Formeln, gegebenenfalls durch Fluor, Chlor und/oder Brom substituiertes, geradkettiges oder verzweigtes Alkyl mit 1 bis 10 Kohlenstoffatomen.

Besonders bevorzugt steht R in den allgemeinen Formeln für gegebenenfalls durch Fluor und/oder Chlor substituiertes, geradkettiges oder verzweigtes Alkyl mit 1 bis 5 Kohlenstoffatomen.

Ganz besonders bevorzugt steht R in den allgemeinen Formeln für t.-Butyl, wobei vorzugsweise die t.-Butyl-Gruppe unsubstituiert ist.

Verwendet man beispielsweise Pivalamidin und Epichlorhydrin als Ausgangsstoffe, so kann der Reaktionsablauf beim erfindungsgemäßen Verfahren durch das folgende Formelschema wiedergegeben werden: 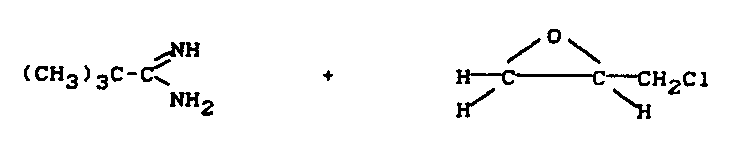 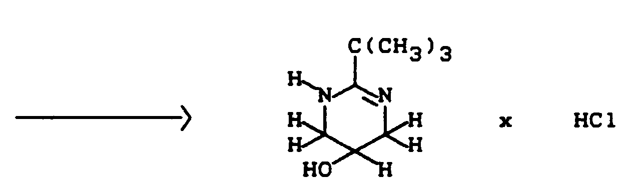
Die beim erfindungsgemäßen Verfahren als Ausgangsstoffe zu verwendenden Amidine der Formel (II) sind bereits bekannt (vergleiche EP-A 320 796).

Das weiter als Ausgangsverbindung einzusetzende Epichlorhydrin der Formel (III) ist eine bekannte Synthesechemikalie.

Das erfindungsgemäße Verfahren wird vorzugsweise unter Verwendung von Verdünnungsmitteln durchgeführt. Als Verdünnungsmittel kommen dabei praktisch alle inerten organischen Lösungsmittel infrage. Hierzu gehören beispielsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlor-benzol, Ether wie Diethyl- und Dibutylether, Glykoldimethylether und Diglykoldimethylether, Tetrahydrofuran und Dioxan, Ketone wie Aceton, Methyl-ethyl-, Methylisopropyl- und Methyl-isobutyl-keton, Ester wie Essigsäuremethylester und -ethylester, Nitrile wie z. B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsäuretriamid.

Als Verdünnungsmittel bevorzugt werden aprotisch polare Solventien, vorzugsweise aus der Reihe der C₁-C₄-Acyl-Nitrile, wie z.B. Acetonitril oder Propionitril, und der Di(C₁-C₄)-Alkyl-Ketone, wie z. B. Aceton, Butanon und Methylisobutylketon.

Die Reaktionstemperaturen können bei dem erfindungsgemäßen Verfahren in einem größeren Bereich variiert werden. Im allgemeinen arbeitet man bei Temperaturen von 0°C bis 150°C, vorzugsweise von 10 bis 120°C und besonders bevorzugt zwischen 20 und 100°C.

Das erfindungsgemäße Verfahren wird im allgemeinen unter Normaldruck durchgeführt. Es ist jedoch auch möglich, unter erhöhtem oder vermindertem Druck - im allgemeinen von 10 hPa bis 10 000 hPa - zu arbeiten.

Bei der Durchführung des erfindungsgemäßen Verfahrens können die Mengen der eingesetzten Ausgangsstoffe ohne wesentliche Nachteile über weite Bereiche variiert werden.

Zur Durchführung des erfindungsgemäßen Verfahrens setzt man vorzugsweise auf 1 Mol Amidin der Formel (II) zwischen 1 und 2 Mol, insbesondere zwischen 1,1 und 1,5 Mol, Epichlorhydrin der Formel (III) ein.

In einer bevorzugten Ausführungsform des erfindungsgemäßen Verfahrens wird das Amidin der Formel (II) in einem Verdünnungsmittel vorgelegt und das Epichlorhydrin wird, gegebenenfalls bei erhöhter Temperatur, vorzugsweise zwischen 20 und 100°C, langsam hinzugefügt. Das Reaktionsgemisch wird dann, im allgemeinen bei erhöhter Temperatur gerührt, bis die Umsetzung abgelaufen ist.

Das Produkt der Formel (I) fällt im allgemeinen direkt kristallin an. Man kann es dann nach Abkühlen durch Absaugen isolieren. Man kann aber auch einengen und das zurückbleibende Produkt durch Behandeln mit einem organischen Lösungsmittel reinigen (vergleiche die Herstellungsbeispiele).

Die nach dem erfindungsgemäßen Verfahren herzustellenden Verbindungen der Formel (I) können als Zwischenprodukte zur Herstellung von Insektiziden verwendet werden (vergleiche EP-A 320 796).

Alle Prozentangaben beziehen sich im vorliegenden Text auf Gewichtsprozente, wenn nichts anderes angegeben ist.

### Herstellungsbeispiele:

### Beispiel 1

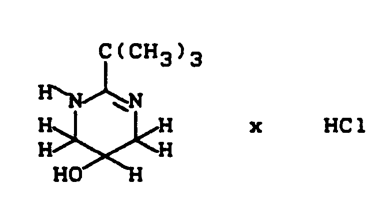

50 g Pivalamidin (81,6 %ig / 0,41 Mol) werden in 300 ml Acetonitril gelöst und nach Erwärmen auf 80°C werden 56 g (0,60 Mol) Epichlorhydrin, gelöst in 50 ml Acetonitril, unter Rühren tropfenweise dazugegeben. Das Reaktionsgemisch wird dann 6 Stunden zum Sieden erhitzt. Nach dem Abkühlen wird eingeengt, der Rückstand mit 100 ml Acetonitril verrührt und das kristalline Produkt durch Absaugen isoliert.
Man erhält 90 g 5-Hydroxy-2-tert-butyl-3,4,5,6-tetrahydro-pyrimidin-Hydrochlorid (83,7 %ig / 96 % der Theorie) in Form farbloser, stark hygroskopischer Kristalle.

### Beispiel 2

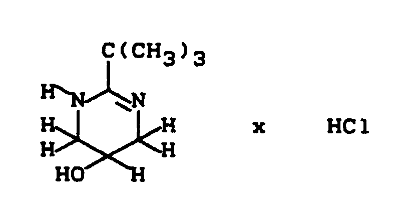

50 g Pivalamidin (81,6 %ig / 0,41 Mol) werden in 300 ml Butanon gelöst und 51 g (0,55 Mol) Epichlorhydrin werden bei 20°C bis 25°C zügig zugetropft. Das Reaktionsgemisch wird 6 Stunden unter Rühren zum Sieden erhitzt und dann wieder abgekühlt. Das kristallin angefallene Produkt wird durch Absaugen isoliert.
Man erhält 62,2 g 5-Hydroxy-2-tert-butyl-3,4,5,6-tetra-hydro-pyrimidin-Hydrochlorid (95 %ig / 76 % der Theorie) in Form farbloser, stark hygroskopischer Kristalle.

## Patentansprüche

1. Verfahren zur Herstellung von 5-Hydroxy-3,4,5,6-tetrahydro-pyrimidin-Derivaten der allgemeinen Formel (I) 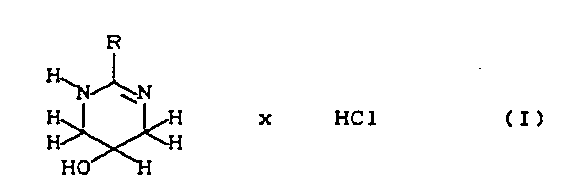 in welcher
R für Alkyl mit 1 bis 20 Kohlenstoffatomen steht, das einen oder mehrere, gleiche oder verschiedene Substituenten aus der Reihe der Halogene enthalten kann,
dadurch gekennzeichnet, daß man Amidine der allgemeinen Formel (II) 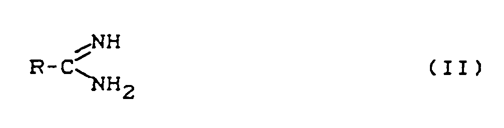 in welcher
R die oben angegebene Bedeutung hat,
mit Epichlorhydrin der Formel (111) 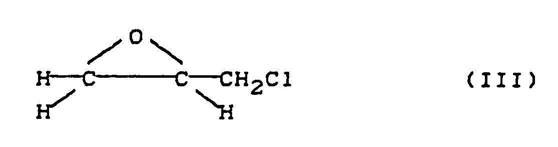 gegebenenfalls in Gegenwart eines Verdünnungsmittels bei Temperaturen zwischen 0°C und 150°C umsetzt, wobei je Mol Amidin der Formel (II) zwischen 1 und 2 Mol des Epichlorhydrins der Formel (III) eingesetzt wird.

2. Verfahren gemäß Anspruch 1, wobei H für gegebenenfalls durch Fluor, Chlor und/oder Brom substituiertes Alkyl mit 1 bis 10 Kohlenstoffatomen steht.

3. Verfahren gemäß Anspruch 1, wobei R für t.-Butyl steht.

4. Verfahren gemäß den Ansprüchen 1 bis 3, wobei ein aprotisch polares Lösungsmittel eingesetzt wird.

5. Verfahren gemäß den Ansprüchen 1 bis 4, wobei das Verfahren bei Temperaturen von 10 bis 120°C durchgeführt wird.

6. Verfahren gemäß den Ansprüchen 1 bis 5, wobei je Mol Amidin der Formel (III) wischen 1,1 und 1,5 Mol Epichlorhydrin eingesetzt wird.

## Claims

1. Process for the preparation of 5-hydroxy-3,4,5,6-tetrahydro-pyrimidine derivatives of the general formula (I) 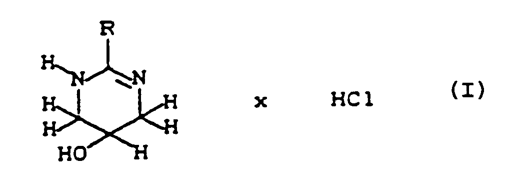 in which
R represents alkyl which has 1 to 20 carbon atoms and can contain one or more identical or different substituents from the series of the halogens,
characterized in that amidines of the general formula (II) 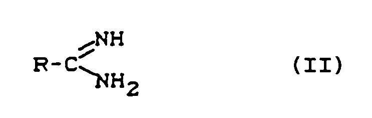 in which
R has the abovementioned meaning,
are reacted with epichlorohydrin of the formula (III) 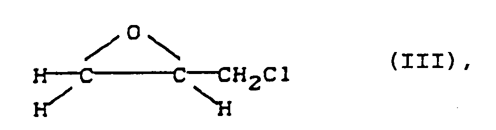 if appropriate in the presence of a diluent at temperatures between 0°C and 150°C, between 1 and 2 moles of epichlorohydrin of the formula (III) being employed per mole of amidine of the formula (II).

2. Process according to Claim 1, where R represents alkyl which has 1 to 10 carbon atoms and is optionally substituted by fluorine, chlorine and/or bromine.

3. Process according to Claim 1, where R represents t.-butyl.

4. Process according to Claims 1 to 3, where an aprotic polar solvent is employed.

5. Process according to Claims 1 to 4, where the process is carried out at temperatures from 10 to 120°C.

6. Process according to Claims 1 to 5, where between 1.1 and 1.5 moles of epichlorohydrin are employed per mole of amidine of the formula (II).

## Revendications

1. Procédé pour la préparation de dérivés de la 5-hydroxy-3,4,5,6-tétrahydropyrimidine, répondant à la formule générale (I) 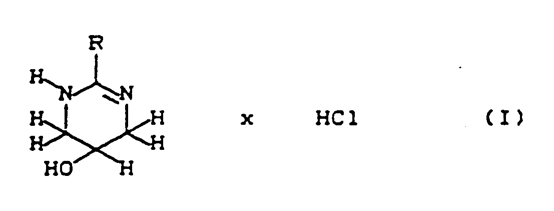 dans laquelle
R représente un groupe alkyle contenant de 1 à 20 atomes de carbone, qui peut contenir un ou plusieurs substituants identiques ou différents choisis parmi la série des halogènes,
caractérisé en ce qu'on fait réagir des amidines répondant à la formule générale (II) 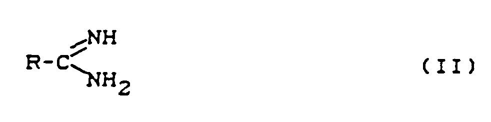 dans laquelle
R a la signification indiquée ci-dessus,
avec une épichlorhydrine de formule (III) 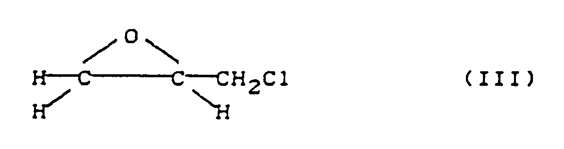 éventuellement en présence d'un diluant, à des températures entre 0°C et 150°C, dans lequel, par mole d'amidine de formule (II), on met en oeuvre entre 1 et 2 moles de l'épichlorhydrine de formule (III).

2. Procédé selon la revendication 1, dans lequel R représente un groupe alkyle contenant de 1 à 10 atomes de carbone, éventuellement substitué par le fluor, le chlore et/ou le brome.

3. Procédé selon la revendication 1, dans lequel R représente un groupe t-butyle.

4. Procédé selon les revendications 1 à 3, dans lequel on met en oeuvre un solvant polaire aprotique.

5. Procédé selon les revendications 1 à 4, dans lequel on effectue le procédé à des températures de 10 à 120°C.

6. Procédé selon les revendications 1 à 5, dans lequel on met en oeuvre, par mole d'amidine de formule (II), entre 1,1 et 1,5 mole d'épichlorhydrine.
